# EUROPEAN PATENT APPLICATION

(11) **EP 4 793 691 A2**
(43) Date of publication of application: **19.08.2026**
(21) Application number: 26158493.2
(22) Date of filing: 13.02.2026
(51) Int. Cl.: G02C 7/08, G02C 11/00

(54) **AN ADAPTIVE EYEWEAR DEVICE AND METHOD FOR REDUCING EYE STRAIN**

(30) Priority: 14.02.2025 FI 20255127
(71) Applicant: IXI Eyewear Oy, 02150 Espoo (FI)
(72) Inventor: Wederhorn, Roosa, 02110 Espoo (FI)
(74) Representative: Moosedog Oy

(57) **Abstract**

Disclosed is an adaptive eyewear device (100) for reducing eye strain, the adaptive eyewear device comprising: an eye-tracking sensor (102A, 102B) operable to monitor the user's gaze and measure a focus distance; a variable-focus lens (110A, 110B) operable to adjust diopter power based on the measured focus distance; a timer (112) operable to track a cumulative duration for which the focus distance remains within a predefined near-work distance range during a predefined time period; and a controller (114) operatively connected to the eye-tracking sensor, the variable-focus lens, and the timer, wherein the controller is operable to: increase the diopter power of the variable-focus lens when the measured focus distance remains within the predefined near-work distance range for at least the predefined time period and decrease the diopter power of the variable-focus lens when the measured focus distance exceeds the predefined near-work distance range or when near work ceases.

## Description

### TECHNICAL FIELD

The present disclosure relates to adaptive eyewear devices. The present disclosure also relates to an adaptive eyewear device and a method for reducing eye strain by use of the adaptive eyewear device.

### BACKGROUND

Focusing on objects closer to eyes for extended periods can potentially strain ciliary muscles of the eyes. When the eyes are not focussed on nearby objects, the ciliary muscles are in a rest/relaxed state. For focussing on a nearby object, the ciliary muscles are required to contract. The contraction is necessary for controlling the shape of lenses of the eyes. Specifically, a curvature or a thickness of a lens needs to increase by dint of the contraction of the ciliary muscles such that bending of light (reflected from the nearby object) and adjustment of focus distance (between the lens of the eyes and the nearby object) is facilitated. An appropriate bending of light and adjustment of the focus distance allows the eyes precisely focus on the nearby object. However, contraction of the ciliary muscles for extended periods of time may strain the ciliary muscles. If the eyes remain focussed on a screen (such as a computer screen or a smartphone) for considerable periods of time, conditions such as digital eye strain or computer vision syndrome may develop. Additional conditions caused by the straining of the ciliary muscles include blurred vision, eye dryness, headache, eye discomfort, eye fatigue, difficulty in refocussing between near and far objects, and so on.

When eyes are focussing on nearby objects, it is recommended to look far away at a distance or focus at distance objects every twenty minutes for a duration of two minutes (or something similar). This allows the ciliary muscle to rest and prevents straining of the eyes. The ciliary muscle and/or a lens (also referring to a natural lens of an eye) only has the ability to contract and, thus, focus on objects at closer distances (from the eyes). When the ciliary muscles are at rest, the eyes do not have the capability to focus on objects at further distances. Therefore, if a user has perfect vison and his/her eyes (i.e., the ciliary muscles of the eyes) are at rest, then a lens providing a positive optical power of two dioptres will facilitate the user to focus on objects at farther distances (such as an object that is 50 centimetres away) while the eyes are at rest. However, using such a lens (providing a positive optical power of two dioptres), the user may not be able to focus on objects at still farther distances (such as an object that is more than 50 centimetres away) and focus on objects that are within 50 centimetres. But, since the eyes are at a rest state, strain on the eyes reduces.

For preventing the ciliary muscles from getting strained, reading glasses or computer glasses may be used. These reduce workload on the ciliary muscles by allowing them to relax and allow the eyes to easily focus on near objects. The reading/computer glasses (lens) provide magnification through adjustment of optical power of the lens. However, an optimum adjustment of power based on focus distance may be highly challenging. Additionally, overcorrection of optical power and usage of inappropriate lenses may cause additional issues.

Conventionally, multifocal lenses provide positive optical power that is designed to be as small as possible. This may accommodate users to focus on a wide range of distances with minimum effort on the part of the eyes. The greater the positive optical power provided, the closer will the user's eyes have a capability to focus with minimum efforts. The lesser the positive optical power provided; greater adaptability will be required from the part of the user's eyes (in terms of contraction of the ciliary muscles).

Therefore, considering the foregoing discussion, there exists a need to overcome the aforementioned drawbacks.

### SUMMARY

The aim of the present disclosure is to provide an adaptive eyewear device and a method for reducing eye strain by use of the adaptive eyewear device. The adaptive eyewear device include components that are able to determine whether a user's eyes are focussing on a nearby object. Based on a determination that the user's eyes are focussing on the nearby object, a power of the adaptive eyewear device is adapted such that the user can focus on the nearby object for extended periods of time without straining the eyes. The adaptive eyewear device facilitates in preventing the user's eyes from getting strained while focussing on nearby objects for significant amounts of time. The with adaptive eyewear device does away with the requirement of having different types of eyewear devices (providing different positive optical powers) for focussing at different distances.

The aim of the present disclosure is achieved by the provided adaptive eyewear device and a method for reducing eye strain by use of the adaptive eyewear device as defined in the appended independent claims to which reference is made to. Advantageous features are set out in the appended dependent claims.

Throughout the description and claims of this specification, the words "*comprise*", "*include*", "*have*", and "*contain*" and variations of these words, for example "*comprising*" and "*comprises*", mean "*including but not limited to*", and do not exclude other components, items, integers, or steps not explicitly disclosed also to be present. Moreover, the singular encompasses the plural unless the context otherwise requires. In particular, where the indefinite article is used, the specification is to be understood as contemplating plurality as well as singularity, unless the context requires otherwise.

### BRIEF DESCRIPTION OF THE DRAWINGS

FIG. 1 illustrates an exemplary adaptive eyewear device that may be used for focussing on near objects while reducing eye strain during the focussing, in accordance with an embodiment of the present disclosure; and
FIG. 2 illustrates steps of a method for reducing eye strain using the adaptive eyewear device, in accordance with an embodiment of the present disclosure.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practising the present disclosure are also possible.

In a first aspect, the present disclosure provides adaptive eyewear device for reducing eye strain, the adaptive eyewear device comprising:
an eye-tracking sensor operable to monitor the user's gaze and measure a focus distance;
a variable-focus lens operable to adjust diopter power based on the measured focus distance;
a timer operable to track a cumulative duration for which the focus distance remains within a predefined near-work distance range; and
a controller operatively connected to the eye-tracking sensor, the variable-focus lens, and the timer, wherein the controller is operable to:
   increase the diopter power of the variable-focus lens if the cumulative duration is equal or exceeds a predefined time period, and
   decrease the diopter power of the variable-focus lens when the measured focus distance exceeds the predefined near-work distance range or when near work ceases.
In other words the controller is operable (configured) to increase the diopter power of the variable-focus lens when the measured focus distance remains within the predefined near-work distance range for at least a predefined time period.

In a second aspect, the present disclosure provides a method for reducing eye strain in an adaptive eyewear device comprising an eye-tracking sensor, a variable-focus lens, a timer, and a controller, the method comprising:
monitoring a user's gaze to measure a focus distance over time, using the eye-tracking sensor;
tracking a cumulative duration for which the measured focus distance remains within a predefined near-work distance range, using the timer; and
adjusting a diopter power of the variable-focus lens, wherein:
   increasing the diopter power when the cumulative duration is equal or greater than a predefined time period, and
   decreasing the diopter power when the measured focus distance exceeds the predefined near-work distance range or when near work ceases.

The present disclosure provides the aforementioned first aspect and the aforementioned second aspect for reducing eye strain caused by focusing on nearby objects (i.e., objects close to the eyes, such as 30-80 cm from the eyes) for extended periods of time. The adaptive eyewear device is adaptive in the sense that an optical power (measured as Diopters) of lenses of the adaptive eyewear device can be adapted or varied. The adaption takes place based on a determination that a user's eyes are focussing on a nearby object(s). The user may be wearing the adaptive eyewear device. The adaptive eyewear device may be worn to prevent the user's eyes from getting strained due to extensive focussing on the nearby object(s) For determining that the user's eyes are focussing on the nearby object(s), the user's gaze is tracked and a focus distance (i.e., a distance between the nearby object(s) and lenses of the adaptive eyewear device worn by the user). If it is determined that the user's eyes are focussing on the nearby object(s) a timer is started to track a cumulative duration for which the focus distance remains within a predefined near-work distance. This way a timer can be used to determine whether the user's eyes have focussed for a predefined period of time. If it is determined that the user's eyes have been focussing on the nearby object(s) for a cumulative duration of time that is greater than the predefined period of time, the optical power of the lenses of the adaptive eyewear device are increased. The increase in the optical power of the lenses of the adaptive eyewear device enables ciliary muscles of the user's eyes to relax. The relaxation of the ciliary muscles is deemed possible as an extent of contraction that is required by the ciliary muscles, to focus on the nearby object(s), is significantly minimized by the dint of the increase in the optical power of the lenses of the adaptive eyewear device. When it is determined, based on the tracking of the user's gaze and the focus distance, that the user's eyes are no longer focussing on the nearby object(s) or if it is determined that a position of the nearby object(s) is such that it is no longer "near", then the increase in the optical power of the lenses of the adaptive eyewear device is reversed.

Throughout the disclosure the term "adaptive eyewear device" refers to an optical device (such as a pair of lenses) that may be worn by the eyes of the user. The adaptive eyewear device includes lenses whose optical power may be automatically adjusted to suit the user's visual needs such as focussing on nearby objects, refocussing on a nearby object, focusing on a distant object after shifting focus from a nearby object, and so on. The adaptive eyewear device has a capability to provide an optimal vision for different focus distances and lighting conditions without a need to switch between other optical devices (such as reading glasses, distance glasses, or computer glasses, progressive glasses). The adaptive eyewear device adjusts lens focus automatically to provide clear vision of an object situated at any focus distance. The automatic adjustment of the lens focus may improve convenience and comfort for users with presbyopia or other conditions. The adaptive eyewear device includes electronic components such as sensors, timers, ad a controller, a battery unit, and so on. The electronic components may require charging which is provided by the battery unit. The battery unit can be charged using a power source when the adaptive eyewear device is in use or when not in use. Examples of the adaptive eyewear device include, but not limited to, electrochromic glasses, liquid crystal glasses, electroactive glasses, smart glasses, and so on.

Throughout the disclosure the term "variable-focus lens" refers to a type of lens included in the adaptive eyewear device whose focal length can be dynamically adjusted. The adjustment facilitates focusing on objects at different focus distances. Thus, the adaptive eyewear device can adapt to different focusing needs of the user and, hence, can function without replacement of the variable-focus lens. The adjustment of the focal length of the variable-focus lens can be fine-tuned for specialized tasks such as performance of near-work whereby the user may be required to work on a computer for an extended period of time. In such scenarios, the focal length or optical power (measured in diopter) of the variable-focus lens can be increased. Examples of the variable-focus lens include, but not limited to, liquid lenses, electroactive lenses, liquid crystal lenses, photochromic lenses, and so on.

Throughout the disclosure the term "eye-tracking sensor" refers to a device that is integrated to the adaptive eyewear device and is operable to monitor and measure gaze direction of the user's eyes, track eye movement, and measure focus distance indicative of a position where the user's eyes are focusing. The focus distance is a distance between an object on which the user's eyes are focusing and the variable-focus lens. The monitoring, measurement, and tracking facilitate adapting the optical power of the adaptive eyewear device (or the variable-focus lens) in real-time and provide optimal visual clarity and comfort while focusing on objects at various distances. The eye-tracking sensor provides seamless switching between near, intermediate, and distance vision.

The eye-tracking sensor may function based on tracking a relative position of the pupil and corneal reflections to determine the gaze direction, tracking pupil dilation and constriction for adapting a tint of the variable-focus lens or adapting light transmission for providing comfort to the user's eyes in varying lighting conditions, measuring an electrical potential generated by eye movements, analyzing of pupil center, iris, or sclera for tracking the gaze direction and position of the user's eyes, capturing and analyzing infrared light reflected from the user's eyes for determining the position and the movement of the user's eyes, recognizing natural eye behaviors for reducing eye strain and enhancing usability, and so on.

Throughout the disclosure the term "timer" is a component included in the adaptive eyewear device that is operable to track a time-period for which the user's eyes have focused on a near object or a distant object. The timer may be triggered based on an input that is indicative of a focus distance measured by the eye-tracking sensor. The timer may be reset based on a change in the focus distance measured by the eye-tracking sensor. The timer may be further operable to track a duration within which an optical power of the variable-focus lens is changed. The tracking ensures smooth transitions when switching between near and far vision. The timer may be implemented using a microcontroller or software. Cumulative duration refers to duration over time which a user has a focus distance within the predefined near-working distance range. The cumulative duration does not need to be continuous, but it can be interrupted for a short period of time. In this scenario, as an example, if user takes a quick look faraway, would not reset the timer but merely would put the timer on pause.

Throughout the disclosure the term "controller" refers to a device that is operatively coupled to the variable-focus lens, the eye-tracking sensor, and the timer. The controller is operable to manage the variable-focus lens by dynamically adjusting the optical power of the variable-focus lens based on inputs (such as gaze direction and/or focus distance) from the eye-tracking sensor. The controller is operable to process data received from the eye-tracking sensor and send signals and commands to the variable-focus lens and the timer. The signals are sent to actuate the variable-focus lens for adjusting the optical power of the variable-focus lens. The commands are sent to trigger the timer or reset the timer.

In accordance with an embodiment, the adaptive eyewear device may include two variable-focus lenses. Each variable-focus lens is worn on each eye. Prior to being worn, it may be necessary to ensure that the battery unit of the adaptive eyewear device is charged. The adaptive eyewear device may include a power button which requires to be pressed for activating the adaptive eyewear device. In some embodiments, the adaptive eyewear device is automatically activated on being worn. Once the adaptive eyewear device is activated, the eye-tracking sensor detects the user's gaze by tracking a position and movement of the user's eyes. Based on the detected user's gaze, a position where the user is looking may be determined. An object may be situated at the determined position (since the user is likely to gaze at the object). The eye-tracking sensor measures a focus distance indicative of a distance between the gazed object and the variable-focus lens.

The eye-tracking sensor may send the focus distance to the controller and the controller may determine whether the focus distance is within a predefined near-work distance range. If the focus distance is within the predefined near-work distance range, the controller determines that the gazed object (on which the eyes of the user is focusing) is a near object. It may be determined that the user is engaged in a work that requires focusing on an object that is closer to the user's eyes (i.e., the variable-focus lens). Optionally, the predefined near-work distance range is from 30 cm (centimetres) up to 80 cm. Thus, the focus distance in the range 30-80 cm and the controller determines that the user is focusing on an object that is within a range of 30-80 cm from the variable-focus lens if the focus distance is within the predefined near-work distance range. The predefined near-work distance range is from 30 cm up to 80 cm. As an example, the predefined near-work distance range may be from 30, 35, 40, 45, 50, or 55 cm up to 50, 55, 60, 65, 70, 75, or 80 cm.

If the focus distance is greater than the predefined near-work distance range, the controller determines that the gazed object (on which the eyes of the user is focusing) is a distant object. In this scenario, the eye-tracking sensor can continue to detect the user's gaze by tracking the position and the movement of the user's eyes. However, in scenarios where the controller determines the focus distance is within the predefined near-work distance range, the controller is operable to trigger the timer. The timer tracks a cumulative duration for which the focus distance is within the predefined near-work distance range (i.e., the eyes of the user are focusing on the near object.

While the timer tracks the cumulative duration for which the focus distance is within the predefined near-work distance range, the controller may determine that a current focus distance is outside the predefined near-work distance range. The determination may be based on an input that is indicative of an updated focus distance. The updated focus distance may be less than 30 cm. Based on the determination, the controller may pause the timer. The controller may be further operable to track a duration for which the timer remains paused. If the controller determines, within a preset time-period after pausing the timer, that the current focus distance is again within the predefined near-work distance range, the controller is operable to restart the timer. The timer, thus, is able to track the cumulative duration for which the focus distance remains within the predefined near-work distance range.

Optionally, the controller is further operable to reset the timer if the focus distance is not in the predefined near-work distance (i.e., outside the predefined near-work distance) for a preset time period. In the context of the present disclosure, the term 'preset time period' refers to a duration that is configured prior to operation and can be tailored based on user preferences or specific application requirements. This preset time period is used to reset the timer when the focus distance remains outside the predefined near-work distance range for a continuous duration. For example, if the focus distance exceeds 80 cm or falls below 30 cm, and the condition persists beyond the preset time period-such as 10 to 20 seconds-the timer is reset to zero. The preset time period offers flexibility, enabling the device to adapt to varying user needs or environmental conditions while ensuring accurate tracking of cumulative focus time. This configurability enhances the device's usability, ensuring smooth operation in diverse scenarios without compromising precision.

The timer is reset to "0" if the updated focus distance remains outside the predefined near-work distance for the preset time period. Thus, if the controller determines that the counter is paused for a duration that is greater than or equal to the preset time period, then the controller resets the timer. However, if the updated focus distance is found to be within the predefined near-work distance range again prior to elapsing of the preset time period, the timer is restarted (instead of being reset). This allows the timer to track the cumulative duration for which the focus distance remains within the predefined near-work distance range. Optionally, the preset time period is from 15 seconds up to 60 seconds.

The controller is operable to determine, based on the tracking of the timer, whether the cumulative duration (for which the focus distance has remained within the predefined near-work distance range) is equal to a predefined time period. If the controller determines that the cumulative duration, for which the focus distance has remained within the predefined near-work distance range, is greater than the predefined time period, then the controller is operable to control the optical power (i.e., diopter power) of the variable-focus lens. The variable-focus lens is operable to adjust the diopter power (of itself) based on the measured focus distance. Specifically, the controller increases the optical power of the variable-focus lens upon determining that the measured focus distance has remained within the near-work distance range for at least the predefined time period (i.e., at least a period equal to the predefined time period).

Optionally, the predefined time period is at least 180 seconds. Thus, if the controller determines that the cumulative duration, for which the focus distance has remained within the predefined near-work distance range, is greater than 180 seconds then the controller is operable to increase the optical power of the variable-focus lens. The predefined time period is at least 180 seconds. As an example, the predefined time period may be from 180, 200, 220, 240, 260, 280, 300, 320, 340, 360, 380 or 400 seconds up to 400, 420, 440, 460, 480, 500, 520, 540, 560, 580, or 600 seconds. While the optical power of the variable-focus lens remains increased, at a certain time-instance the controller may determine, based on an input from the eye-tracking sensor, that the focus distance has exceeded the predefined near-work distance range. Such determination may take place when the eye-tracking sensor measures the current focus distance and sends the current focus distance as the input. The current focus distance measured at the time-instance may be greater than 80 cm.

This determination indicates that a gazed object (on which the eyes of the user is focusing on at the time-instance) is a distant object and that the user is no-longer involved in the work requiring focusing on a near object. Based on the determination that the focus distance has exceeded the predefined near-work distance range (i.e., the focus distance is greater than 80cm), the controller is operable to decrease the optical power (i.e., the diopter power) of the variable-focus lens. It is to be noted that in each scenario, where the optical power of the variable-focus lens is increased or decreased, the increase/decrease is gradual.

The adjustment of the diopter power by the variable-focus lens is inversely proportional to the measured focus distance, ensuring smooth and precise transitions for the user's visual comfort. Specifically, when the focus distance decreases within the predefined range of 30 cm to 80 cm, the diopter power increases proportionally. At a focus distance of 80 cm, the diopter power is set to 1.25 diopters. Conversely, when the focus distance increases and exceeds the predefined near-work distance range of 80 cm, the diopter power decreases proportionally, following the same rate of adjustment. This gradual and proportional increase or decrease ensures seamless visual transitions, preventing sudden shifts in focus that could cause eye strain or discomfort. The system is designed to respond dynamically to changes in focus distance, maintaining optimal visual clarity without abrupt interruptions.

Optionally, the controller is further operable to adjust rate of increase or decrease in diopter power gradually over a set transition time. In the context of the present disclosure, the term 'gradually' refers to a smooth and incremental adjustment of the diopter power of the variable-focus lens. The gradual adjustment ensures a seamless adaptation of the user's vision to the changing focus distance, thereby preventing discomfort, such as headaches or visual disorientation, which may occur with abrupt optical power changes. A gradual variation of the optical power of the variable-focus lens may protect the user's eyes from experiencing discomfort disorientation (such as eye strain, headaches, temporary blurred vision, and so on) or temporary vision disruption. Furthermore, the gradual variation of the optical power of the variable-focus lens provides a comfortable and a seamless user experience. The set transition time may be in certain milliseconds.

Optionally, the adaptive eyewear device further comprises a flicker sensor that is operable to detect ambient flickering lighting conditions and monitor flickering. The controller is operable to adjust the diopter power based on the detected flicker. The controller is operatively connected to the flicker sensor. The flicker sensor detects fluctuations in light intensity in the ambience where the user, wearing the adaptive eyewear device, is present. The flicker sensor may include a light-sensitive element that measured light intensity over a time period and captures variations in various parameters (such as amplitude or frequency) of the lighting levels in the ambience. The flickering lighting conditions can be detected when the ambience includes artificial light sources such as fluorescent lamps or light emitting diode (LED) lighting. Once the flickering lighting conditions are detected, the flicker sensor sends an input to the controller indicating that flickering lighting conditions have been detected.

Furthermore, it may be detected, based on gaze detection by the eye-tracking sensor, that the user is focusing on a screen that is emitting artificial light. The user may be involved in a work that requires focusing on the screen emitting artificial light. In this scenario, the flicker sensor is operable to monitor flickering on the screen (which may be caused by sudden changes in brightness of the screen). When a flickering is detected on the screen, the flicker sensor sends an input to the controller indicating that flickering has been detected on the screen.

Based on the received inputs, the controller adjusts the optical power of the variable-focus lens. Specifically, if the input indicates that a flicker has been detected on the screen, then the controller determines that the user is engaged in a work whereby the user's eyes are focusing on a near object, i.e., the screen. In this scenario, the controller may trigger the timer. If the controller determines that a focus distance has remained within the predefined near-work distance range during the predefined time period (which starts at the instant when the timer is triggered), then the optical power (i.e., the diopter power) of the variable-focus lens is increased.

The present disclosure also relates to the second aspect as described above. Various embodiments and variants disclosed above, with respect to the aforementioned first aspect, apply *mutatis mutandis* to the second aspect.

Optionally, the predefined near-work distance range is from 30 cm up to 80 cm.

Optionally, the predefined time period is at least 180 seconds up to 600 seconds.

Optionally, the method further comprises further comprises adjusting a rate of increase or decrease in diopter power gradually over a set transition time. The effect of this gradual increase of optical power is that the change becomes non-noticeable for the user. This is because the human eye naturally accommodates to slow dioptric changes in the same way it accommodates to changes in focus distance. The benefit of the gradual transition is that the status of the adaptive lenses can change over from the standard near-work mode to the strain-releaving near-work mode without causing interference for the user. The rate of increase or decrease is preferably 0.5D in 30 seconds. As an further example the rate of increase or decrease can be 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9 or 2.0 D in 10, 15, 20, 25, 30, 35, 40, 45, 50, 55 or 60 seconds.

Optionally, the method further comprises further comprises resetting the timer if the focus distance is not in the predefined near-work distance for a preset time period.

Optionally, the preset time period is from 15 seconds up to 60 seconds.

Optionally, the method further comprises further comprises adjusting the diopter power based on a detected flicker of ambient flickering light conditions.

### DETAILED DESCRIPTION OF THE DRAWINGS

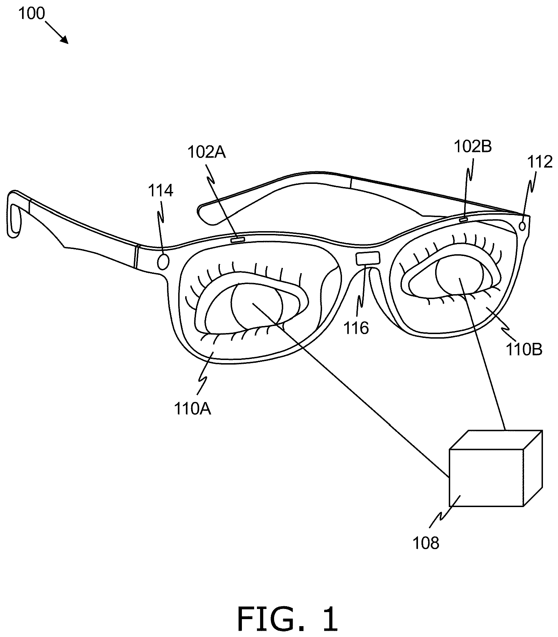

Referring to FIG. 1, there is illustrated an exemplary adaptive eyewear device **100** that may be used for focussing on near objects **108** while reducing eye strain during the focussing, in accordance with an embodiment of the present disclosure. The adaptive eyewear device **100** includes a pair of eye-tracking sensors that comprises a first eye-tracking sensor **102A** and a second eye-tracking sensor **102B,** a pair of variable-focus lenses that comprises a first variable-focus lens **110A** and a second variable-focus lens **110B,** a timer **112,** and a controller **114.**

Additionally, the adaptive eyewear device **100** may include a flicker sensor **116.** The first eye-tracking sensor **102A** measures direction of gaze of a first eye and the second eye-tracking sensor **102B** measures direction of gaze of the second eye. The gaze direction information of each eye is processed by the controller **114** is used to determine focus distance. The first variable-focus lens **110A** may be worn on a right eye of the user and the second variable-focus lens **110B** may be worn on a left eye of the user.

Each of the first eye-tracking sensor **102A** and the second eye-tracking sensor **102B** are operable to monitor the user's gaze and measure a focus distance. Each of the first variable-focus lens **110A** and the second variable-focus lens **110B** are operable to adjust respective diopter power (optical power) based on the measured focus distances. The timer **112** is operable to track a cumulative duration for which the focus distance remains within a predefined near-work distance range during a predefined time period.

The controller **114** is operatively connected to the first eye-tracking sensor **102A,** the second eye-tracking sensor **102B,** the first variable-focus lens **110A,** the second variable-focus lens **110B,** and the timer **112.** The controller **114** is operable to increase the diopter power of each of the first variable-focus lens **110A** and the second variable-focus lens **110B** when the measured focus distances remain within the predefined near-work distance range for at least the predefined time period. The controller **114** is further operable to decrease the diopter power of each of the first variable-focus lens **110A** and the second variable-focus lens **110B** when the measured focus distance exceeds the predefined near-work distance range or when near work ceases.

FIG. 1 is merely an example, which should not unduly limit the scope of the claims herein. A person skilled in the art will recognize many variations, alternatives, and modifications of embodiments of the present disclosure.

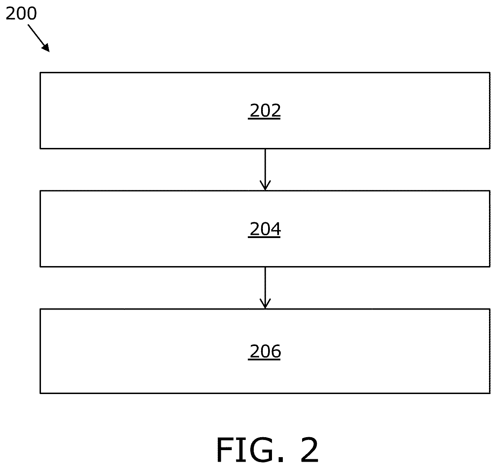

Referring to FIG. 2, depicted are steps of a method **200** for reducing eye strain using the adaptive eyewear device **100,** in accordance with an embodiment of the present disclosure. The method **200** is implemented in the controller **114.** At step **202,** a user's gaze is monitored to measure a focus distance over time, using the eye-tracking sensor **102.** At step **204,** a cumulative duration, for which the measured focus distance remains within a predefined near-work distance range, is tracked using the timer **112.** At step **206,** a diopter power (i.e., an optical power) of the variable-focus lens **110** is adjusted. The diopter power (i.e., optical power) is increased when the measured focus distance is within the predefined near-work distance range for at least a predefined time period. The diopter power (i.e., optical power) is decreased when the measured focus distance exceeds the predefined near-work distance range or when near work ceases.

The aforementioned steps are only illustrative, and other alternatives can also be provided where one or more steps are added, one or more steps are removed, or one or more steps are provided in a different sequence without departing from the scope of the claims herein.

## Claims

1. An adaptive eyewear device (100) for reducing eye strain, the adaptive eyewear device comprising:
• an eye-tracking sensor (102A, 102B) operable to monitor the user's gaze and measure a focus distance;
• a variable-focus lens (110A, 110B) operable to adjust diopter power based on the measured focus distance;
• a timer (112) operable to track a cumulative duration for which the focus distance remains within a predefined near-work distance range; and
• a controller (114) operatively connected to the eye-tracking sensor, the variable-focus lens, and the timer, wherein the controller is operable to:
∘ increase the diopter power of the variable-focus lens if the cumulative duration is equal or exceeds a predefined time period; and
∘ decrease the diopter power of the variable-focus lens when the measured focus distance exceeds the predefined near-work distance range or when near work ceases.

2. An adaptive eyewear device (100) of claim 1, wherein the predefined near-work distance range is from 30 cm up to 80 cm.

3. An adaptive eyewear device (100) of claims 1 or 2, wherein the predefined time period is at least 180 seconds.

4. An adaptive eyewear device (100) according to any of the preceding claims, wherein the controller (114) is further configured to adjust rate of increase or decrease in diopter power gradually over a set transition time.

5. An adaptive eyewear device (100) according to any of the preceding claims, wherein the controller is further operable to reset the timer if the focus distance is not in the predefined near-work distance for a preset time period.

6. An adaptive eyewear device (100) according to claim 5, wherein the preset time period is from 15 seconds up to 60 seconds.

7. An adaptive eyewear device (100) according to any of the preceding claims, further comprising a flicker sensor operable to detect ambient flickering lighting conditions and monitor flickering; and wherein the controller (114) is operable to adjust the diopter power based on the detected flicker.

8. A method of reducing eye strain in an adaptive eyewear device (100) comprising an eye-tracking sensor (102A, 102B), a variable-focus lens (110A, 110B), a timer (112), and a controller (114), the method comprising:
- monitoring a user's gaze to measure a focus distance over time, using the eye-tracking sensor;
- tracking a cumulative duration for which the measured focus distance remains within a predefined near-work distance range, using the timer; and
- adjusting a diopter power of the variable-focus lens, wherein:
increasing the diopter power when the cumulative duration is equal or greater than a predefined time period, and
- decreasing the diopter power when the measured focus distance exceeds the predefined near-work distance range or when near work ceases.

9. A method according to claim 8, wherein the predefined near-work distance range is from 30 cm up to 80 cm.

10. A method according to claims 8 or 9, wherein the predefined time period is at least 180 seconds.

11. A method according to any of the claims 8-10, further comprising adjusting a rate of increase or decrease in diopter power gradually over a set transition time.

12. A method according to any of the claims 8-11, further comprising resetting the timer if the focus distance is not in the predefined near-work distance for a preset time period.

13. A method according to claim 12, wherein the preset time period is from 15 seconds up to 60 seconds.

14. A method according to any of the claims 8-13, further comprising adjusting the diopter power based on a detected flicker of ambient flickering light conditions.
